(19) Europäisches Patentamt
European Patent Office
Office européen des brevets

(11) **EP 4 206 268 A1**

(12) **EUROPEAN PATENT APPLICATION**
published in accordance with Art. 153(4) EPC

(43) Date of publication:
**05.07.2023 Bulletin 2023/27**

(21) Application number: **22846147.1**

(22) Date of filing: **14.07.2022**

(51) International Patent Classification (IPC):
*C08J 11/24* (2006.01)   *C07C 63/26* (2006.01)
*C07C 51/09* (2006.01)   *C07C 51/42* (2006.01)
*C08G 63/181* (2006.01)   *C08G 63/183* (2006.01)
*C08K 5/00* (2006.01)   *C08K 5/10* (2006.01)

(52) Cooperative Patent Classification (CPC):
**C07C 51/09; C07C 51/42; C07C 63/26;
C08G 63/181; C08G 63/183; C08J 11/24;
C08K 5/00; C08K 5/10; Y02W 30/62**

(86) International application number:
**PCT/KR2022/010302**

(87) International publication number:
**WO 2023/003277 (26.01.2023 Gazette 2023/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.07.2021   KR 20210094470
19.07.2021   KR 20210094471
19.07.2021   KR 20210094472
19.07.2021   KR 20210094473**

(71) Applicant: **Lg Chem, Ltd.
Seoul 07336 (KR)**

(72) Inventors:
• **PARK, Tae Seung**
**Daejeon 34122 (KR)**
• **KIM, Jeongnam**
**Daejeon 34122 (KR)**
• **HWANG, Jihwan**
**Daejeon 34122 (KR)**
• **HONG, Mooho**
**Daejeon 34122 (KR)**

(74) Representative: **Goddar, Heinz J.
Boehmert & Boehmert
Anwaltspartnerschaft mbB
Pettenkoferstrasse 22
80336 München (DE)**

(54) **MONOMER COMPOSITION FOR SYNTHESIZING RECYCLED PLASTIC, METHOD FOR MANUFACTURING SAME, AND RECYCLED PLASTIC, MOLDED ARTICLE, AND PLASTICIZER COMPOSITION USING SAME**

(57)    The present invention relates to a monomer composition for synthesizing recycled plastic which is capable of recovering from a (co)polymer synthesized from a monomer containing terephthalic acid, extremely reducing the content of isophthalic acid, which is an impurity, and securing terephthalic acid having increased particle size, a method for preparing the same, and a recycled plastic, molded product, and plasticizer composition using the same.

EP 4 206 268 A1

**Description**

[TECHNICAL FIELD]

CROSS-REFERENCE TO RELATED APPLICATION(S)

This application claims the benefit of Korean Patent Application No. 10-2021-0094470 filed on July 19, 2021, Korean Patent Application No. 10-2021-0094471 filed on July 19, 2021, Korean Patent Application No. 10-2021-0094472 filed on July 19, 2021 and Korean Patent Application No. 10-2021-0094473 filed on July 19, 2021 in the Korean Intellectual Property Office, the contents of which are incorporated herein by reference in their entirety.

The present invention relates to a monomer composition for synthesizing recycled plastic that is capable of remarkably reducing the content of isophthalic acid, which is an impurity, and thus increasing the purity of terephthalic acid, when recovering terephthalic acid through a depolymerization reaction of a (co)polymer synthesized from a monomer containing terephthalic acid, and also of securing terephthalic acid having increased particle size through recrystallization, a method for preparing the same, and a recycled plastic, molded product, and plasticizer composition using the same.

[BACKGROUND ART]

Polyethylene terephthalate (PET) is a thermoplastic (co)polymer that has excellent characteristics such as excellent transparency and heat insulation property, and is a plastic widely used in electric wire coverings, daily necessities, toys, electrical insulators, radios, television cases, packaging materials, and the like.

Although polyethylene terephthalate is widely used for various purposes, environmental and health concerns during waste treatment have been continuously raised. Currently, a physical recycling method is being carried out, but in this case, a problem accompanying the deterioration of the quality occurs, and thus, research on the chemical recycling of polyethylene terephthalate are underway.

Terephthalic acid is a useful compound used as a raw material for various kinds of products, which is used as a main raw material for polyethylene terephthalate (PET), polyester fibers, and polyester films for packaging and containers.

Subjecting ethylene glycol together with terephthalic acid to a polycondensation to produce polyethylene terephthalate (PET) is a reversible reaction process, and polyethylene terephthalate (PET) can be depolymerized and recycled to a monomer or oligomer.

Various methods have been conventionally proposed as a method for decomposing polyethylene terephthalate (PET) to recover a monomer as a raw material. Monomers that can be used in the polycondensation reaction for reproducing into a recycled plastic can be obtained through alkali decomposition of polyethylene terephthalate (PET) waste.

For example, polyethylene terephthalate (PET) decomposition products under basic conditions include salts of ethylene glycol and terephthalic acid, and a salt of terephthalic acid is further subjected to a neutralization reaction with a strong acid to prepare terephthalic acid.

However, terephthalic acid obtained by the existing method usually contains 1 to 2% isophthalic acid as an impurity, and thus, when it is reused as a raw material for producing high value-added plastics such as PBT/TPEE, there is a limit in that problems of deterioration of (co)polymer physical properties (low melting point, low tensile strength, low stiffness, etc.) occur.

Therefore, in the process of decomposing the (co)polymer synthesized from the monomer containing terephthalic acid including polyethylene terephthalate (PET) and recovering the monomer as a raw material, there is a need to develop a method that can significantly reduce the content of isophthalic acid which is an impurity.

[DETAILED DESCRIPTION OF THE INVENTION]

[Technical Problem]

It is an object of the present invention to provide a monomer composition for synthesizing recycled plastic that is capable of remarkably reducing the content of isophthalic acid, which is an impurity, and thus increasing the purity of terephthalic acid, when recovering terephthalic acid through a depolymerization reaction of a (co)polymer synthesized from a monomer including terephthalic acid, and also of securing terephthalic acid having increased particle size through recrystallization.

It is another object of the present invention to provide a method for preparing a monomer composition for synthesizing recycled plastic, and a recycled plastic, molded product, and plasticizer composition using the monomer composition for synthesizing recycled plastic.

[Technical Solution]

In order to achieve the above object, provided herein is a monomer composition for synthesizing recycled plastic, which comprises terephthalic acid, and which further comprises isophthalic acid having a molar ratio of less than 0.5 mol% based on 100 mol% of the monomer composition for synthesizing recycled plastic, wherein the terephthalic acid has a particle size of 10 $\mu$m or more and 300 $\mu$m or less, and wherein the monomer composition for synthesizing recycled plastic is recovered from a (co)polymer synthesized from a monomer containing terephthalic acid.

Also provided herein is a method for preparing a monomer composition for synthesizing recycled plastic, comprising the steps of: subjecting a (co)polymer synthesized from a monomer containing terephthalic acid to a depolymerization reaction and removing a diol component; dissolving the depolymerization reaction product from which the diol component has been removed in an organic solvent and then recrystallizing the product; and washing the result of the recrystallization step with a solvent at a temperature of 200°C or more and 300°C or less.

Further provided herein is a recycled plastic comprising a reaction product of the monomer composition for synthesizing recycled plastic and a comonomer.

Further provided herein is a molded product comprising the recycled plastic. Further provided herein is a plasticizer composition comprising a reaction product of the monomer composition for synthesizing recycled plastic and an alcohol.

Below, a monomer composition for synthesizing recycled plastic, a method for preparing the same, and a recycled plastic, molded product, and plasticizer composition using the same according to specific embodiments of the present invention will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.


1. Monomer Composition for Synthesizing Recycled Plastic

According to one embodiment of the present invention, there can be provided a monomer composition for synthesizing recycled plastic, which comprises terephthalic acid, and which further comprises isophthalic acid having a molar ratio of less than 0.5 mol% based on 100 mol% of the monomer composition for synthesizing recycled plastic, wherein the terephthalic acid has a particle size of 10 $\mu$m or more and 300 $\mu$m or less, and wherein the monomer composition for synthesizing recycled plastic is recovered from a (co)polymer synthesized from a monomer containing terephthalic acid.

The present inventors has found through experiments that, despite that it is recovered from a (co)polymer synthesized from a monomer containing terephthalic acid, as in the monomer composition for synthesizing recycled plastic of the above one embodiment, the ratio of isophthalic acid, which is other monomer and not terephthalic acid, which is the main synthetic target material in the present invention, is extremely reduced to less than 0.5 mol% based on 100 mol% of the total monomer compound contained in the monomer composition for synthesizing recycled plastic, thereby capable of realizing excellent physical properties in the synthesis of polyethylene terephthalate or high value-added plastics (PBT, TPEE), and completed the present invention.

The inventors also confirmed that the particle size of terephthalic acid is increased to 10 $\mu$m or more and 300 $\mu$m or less as compared to the conventional one, so that more terephthalic acid is recovered from the (co)polymer synthesized from monomers containing terephthalic acid to minimize the loss of terephthalic acid, thereby increasing the recovery efficiency.

In particular, terephthalic acid recovered from a (co)polymer synthesized from a monomer containing terephthalic acid obtained by a conventionally known method usually contains 1 to 2% isophthalic acid as an impurity, or contains an organic solvent used for recrystallization as an impurity, whereas in the present invention, isophthalic acid or the organic solvent used for recrystallization could be almost completely removed by the recrystallization step and the high-temperature washing step which are characteristic in the method for preparing the monomer composition for synthesizing recycled plastics, which will be described later.

Specifically, the monomer composition for synthesizing recycled plastic of the one embodiment may include terephthalic acid. The terephthalic acid is characterized by being recovered from the (co)polymer synthesized from the monomer containing terephthalic acid which is used for the recovery of the monomer composition for synthesizing recycled plastic.

That is, this means that recovery is performed from the (co)polymer synthesized from the monomer containing terephthalic acid in order to obtain the monomer composition for synthesizing recycled plastic of the one embodiment, and as a result, terephthalic acid is also obtained together. Therefore, apart from the recovery from the (co)polymer synthesized from the monomer containing terephthalic acid in order to prepare the monomer composition for synthesizing recycled plastic of the one embodiment, the case where new terephthalic acid is added from the outside is not included in the scope of terephthalic acid of the present invention.

Specifically, "recovered from the (co)polymer synthesized from the monomer containing terephthalic acid" means that it is obtained through a depolymerization reaction of the (co)polymer synthesized from the monomer containing terephthalic acid.

More specifically, the depolymerization reaction can be carried out under acidic, neutral, or basic conditions. For example, when the depolymerization reaction proceeds under basic (alkaline) conditions, terephthalic acid salt called $Na_2$-TPA and ethylene glycol are primarily produced from the polyethylene terephthalate, and $Na_2$-TPA is converted to TPA through a secondary strong acid neutralization, thereby capable of recovering terephthalic acid.

That is, the terephthalic acid recovered from the (co)polymer synthesized from the monomer containing terephthalic acid may include a basic (alkaline) decomposition product of the (co)polymer synthesized from the monomer containing terephthalic acid, an acid-neutralized product thereof, or a mixture thereof. Specifically, the basic (alkaline) decomposition product of the (co)polymer synthesized from the monomer containing terephthalic acid may include $Na_2$-TPA, and the acid-neutralized product of the basic (alkaline) decomposition product of the (co)polymer synthesized from the monomer containing terephthalic acid may include terephthalic acid.

The terephthalic acid may have a molar ratio of more than 99.5 mol%, or 99.9 mol% or more, or more than 99.5 mol% and 100 mol% or less, or 99.9 mol% or more and 100 mol% or less, based on 100 mol% of the total monomer compounds contained in the monomer composition for synthesizing recycled plastic.

Examples of the method for measuring the molar ratio of terephthalic acid are not particularly limited, and, for example, $^1$H NMR, ICP-MS analysis, HPLC analysis, and the like can be used without limitation. As the specific methods, conditions, devices and the like of the NMR, ICP-MS and HPLC, conventionally well-known various contents can be applied without limitation.

In an example of the method for measuring the molar ratio of terephthalic acid, 5 mg or more and 20 mg or less of the monomer composition for synthesizing recycled plastic is collected as a sample under the conditions of normal pressure, 20°C or more and 30°C or less, dissolved in 1 ml of DMSO-d6 solvent, and then $^1$H NMR spectrum is obtained via an Agilent DD1 500 MHz NMR instrument, and by using the analysis software (MestReC), the detected peaks of all materials such as terephthalic acid (TPA), isophthalic acid (IPA) and the like are respectively assigned, integrated, and the molar ratio (mol%) of terephthalic acid contained in 100 mol% of the total monomer compounds analyzed in the sample is calculated based on the integral values of the peaks.

In this manner, the ratio of terephthalic acid, which is the main synthetic target material in the present invention, is extremely increased to more than 99.5 mol% based on 100 mol% of the total monomer compounds contained in the monomer composition for synthesizing recycled plastic, and terephthalic acid or impurity monomers (e.g., isophthalic acid) are minimized, thereby capable of realizing excellent physical properties in the synthesis of polyethylene terephthalate or high value-added plastics (PBT, TPEE).

In particular, in the monomer composition for synthesizing recycled plastic of the one embodiment, the molar ratio of terephthalic acid being extremely increased to more than 99.5 mol% based on 100 mol% of the total monomer compounds contained in the monomer composition for synthesizing recycled plastic is because, through the step of dissolving the depolymerization reaction product in an organic solvent and then recrystallizing the product as described later, and the step of washing the result of the recrystallization step with a solvent at a temperature of 200°C or more and 300°C or less, the solubility of terephthalic acid or its salt is increased and thus crystals, or impurities such as isophthalic acid interposed between crystals can be dissolved with a solvent to the maximum, and further, the temperature can be lowered using a difference of solubility between the dissolved terephthalic acid and the impurities, thereby recovering terephthalic acid again.

In the monomer composition for synthesizing recycled plastic of the one embodiment, when the molar ratio of terephthalic acid is reduced to 99.5 mol% or less based on 100 mol% of the total monomer compounds contained in the monomer composition for synthesizing recycled plastic, it is difficult to remove a sufficient level of impurities and secure high purity and high yield of terephthalic acid, whereby the terephthalic acid recovery process efficiency of terephthalic acid decreases, and the physical properties of the recovered terephthalic acid and the (co)polymer synthesized therefrom may be deteriorated.

Further, the monomer composition for synthesizing recycled plastic of the one embodiment may further include isophthalic acid having a molar ratio of less than 0.5 mol% based on 100 mol% of the total monomer compounds contained in the monomer composition for synthesizing recycled plastic.

The isophthalic acid is characterized by being recovered from the (co)polymer synthesized from the monomer containing terephthalic acid that is used for the recovery of the monomer composition for synthesizing recycled plastic.

That is, this means that recovery is performed from the (co)polymer synthesized from the monomer containing terephthalic acid in order to obtain the monomer composition for synthesizing recycled plastic of the one embodiment, and as a result, terephthalic acid is also obtained together. Therefore, apart from the recovery from the (co)polymer synthesized from the monomer containing terephthalic acid in order to prepare the monomer composition for synthesizing recycled plastic of the one embodiment, the case where new terephthalic acid is added from the outside is not included in the scope of terephthalic acid of the present invention.

Specifically, "recovered from the (co)polymer synthesized from the monomer containing terephthalic acid" means that it is obtained through a depolymerization reaction of the (co)polymer synthesized from the monomer containing terephthalic acid. The depolymerization reaction can be carried out under acidic, neutral or basic conditions, and particularly, the depolymerization reaction can proceed under basic (alkaline) conditions.

The terephthalic acid may have a molar ratio of less than 0.5 mol%, or 0.1 mol% or less, or 0 mol% or more and less than 0.5 mol%, or 0 mol% or more and 0.1 mol% or less, based on 100 mol% of the total monomer compounds contained in the monomer composition for synthesizing recycled plastic.

Examples of the method for measuring the molar ratio of terephthalic acid are not particularly limited, and, for example, $^1$H NMR, ICP-MS analysis, HPLC analysis, and the like can be used without limitation. As for the specific methods, conditions, devices and the like of the NMR, ICP-MS and HPLC, conventionally well-known various contents can be applied without limitation.

In one example of the method for measuring the molar ratio of terephthalic acid, 5 mg or more and 20 mg or less of the monomer composition for synthesizing recycled plastic is collected as a sample under the conditions of normal pressure, 20°C or more and 30°C or less, dissolved in 1 ml DMSO-d6 solvent, and then $^1$H NMR spectrum is obtained via an Agilent DD1 500 MHz NMR instrument, and by using the analysis software (MestReC), the detected peaks of all materials such as terephthalic acid (TPA), isophthalic acid (IPA) and the like are respectively assigned, integrated, and the molar ratio (mol%) of terephthalic acid contained in 100 mol% of all the monomer compounds analyzed in the sample is calculated based on the integral values of the peaks.

In this manner, the ratio of terephthalic acid, which is the main synthetic target material in the present invention, is extremely decreased to 0.5 mol% or less based on 100 mol% of the total monomer compounds contained in the monomer composition for synthesizing recycled plastic, thereby capable of realizing excellent physical properties in the synthesis of polyethylene terephthalate or high value-added plastics (PBT, TPEE).

In particular, in the monomer composition for synthesizing recycled plastic of the one embodiment, the molar ratio of terephthalic acid being extremely decreased to 0.5 mol% or less based on 100 mol% of the total monomer compounds contained in the monomer composition for synthesizing recycled plastic is because, through the step of washing the result of the recrystallization step with a solvent at a temperature of 200°C or more and 300°C or less as described later, the solubility of terephthalic acid or its salt is increased and thus crystals, or impurities such as isophthalic acid interposed between crystals can be dissolved with a solvent to the maximum, and further, the temperature can be lowered using a difference of solubility between the dissolved terephthalic acid and the impurities, thereby recovering terephthalic acid again.

In the monomer composition for synthesizing recycled plastic of the one embodiment, when the molar ratio of terephthalic acid is increased to 0.5 mol% or more based on 100 mol% of the total monomer compounds contained in the monomer composition for synthesizing recycled plastic, it is difficult to remove a sufficient level of impurities and secure high purity and high yield of terephthalic acid, whereby the recovery process efficiency of terephthalic acid decreases, and the physical properties of the recovered terephthalic acid and the (co)polymer synthesized therefrom may be deteriorated.

Further, the monomer composition for synthesizing recycled plastic of the one embodiment is characterized by being recovered from a (co)polymer synthesized from a monomer containing terephthalic acid. That is, this means that recovery is performed from the (co)polymer synthesized from the monomer containing terephthalic acid in order to obtain the monomer composition for synthesizing recycled plastic of the one embodiment, and as a result, a monomer composition for synthesizing recycled plastic containing terephthalic acid and isophthalic acid is obtained together.

In the (co)polymer synthesized from the monomer containing terephthalic acid, the (co)polymer includes both a polymer and a copolymer, and collectively refers to a reaction product obtained from the (co)polymerization of monomers. The (co)polymer may include all low molecular weight compounds, oligomers, and polymers depending on the molecular weight range.

The (co)polymer synthesized from the monomer containing terephthalic acid may include at least one (co)polymer selected from the group consisting of polyalkylene terephthalate, polyalkylene terephthalate-based copolymer, and thermoplastic polyester elastomer. That is, the (co)polymer synthesized from the monomer containing terephthalic acid may include one type of polyalkylene terephthalate, one type of polyalkylene terephthalate-based copolymer, one type of thermoplastic polyester elastomer, or a mixture of two or more thereof.

The polyalkylene terephthalate-based copolymer means a copolymer obtained by further reacting an additional comonomer based on alkylene glycol and terephthalic acid which are monomers for synthesizing polyalkylene terephthalate.

The (co)polymer synthesized from the monomer containing terephthalic acid may include a reaction product of terephthalic acid and a comonomer. That is, the monomer containing terephthalic acid may further include a comonomer together with terephthalic acid.

Examples of the comonomer capable of reacting with terephthalic acid are also not particularly limited, and specific examples thereof may include aliphatic diols, polyalkylene oxides, fatty acids, fatty acid derivatives, or combinations thereof.

As the aliphatic diol, for example, a diol having a number average molecular weight (Mn) of 300 g/mol or less, i.e., at least one selected among ethylene glycol, propylene glycol, 1,2-propanediol, 1,3-propanediol, 1,4-butanediol (1,4-BG), 1,5-pentanediol, 1,6-hexanediol, 1,4-cyclohexanedimethanol (1,4-CHDM) can be used. As a specific example, 1,4-butanediol, ethylene glycol, 1,4-cyclohexanedimethanol, or a mixture thereof can be used.

The polyalkylene oxide is a unit constituting a soft fraction, and may include an aliphatic polyether as a constituent component. As an example, at least one selected among polyoxyethylene glycol, polypropylene glycol, poly(tetramethylene ether)glycol (PTMEG), polyoxyhexamethylene glycol, a copolymer of ethylene oxide and propylene oxide, an ethylene oxide addition polymer of polypropylene oxide glycol, and a copolymer of ethylene oxide and tetrahydrofuran can be used, and as a specific example, PTMEG can be used. In particular, PTMEG having a number average molecular weight (Mn) of 600 g/mol to 3,000 g/mol, 1,000 g/mol to 2,500 g/mol, or 1,500 g/mol to 2,200 g/mol can be used.

The fatty acid may be at least one type of aliphatic carboxylic acid compounds excluding terephthalic acid as an example, and adipic acid can be used as a specific example. The fatty acid derivative is a compound derived from the above-mentioned fatty acid, and as an example, at least one selected among fatty acid ester, fatty acid chloride, fatty acid anhydride, and fatty acid amide can be used, and as a specific example, adipic acid ester can be used.

In a more specific example, when 1,4-butanediol which is the aliphatic diol is used as a comonomer capable of reacting with the terephthalic acid, polybutylene terephthalate (PBT), which is a type of polyalkylene terephthalate, can be obtained by polymerization reaction of terephthalic acid and 1,4-butanediol.

Further, when ethylene glycol, which is the aliphatic diol, is used as a comonomer capable of reacting with the terephthalic acid, polyethylene terephthalate (PET), which is a type of the polyalkylene terephthalate, can be obtained through a polymerization reaction of terephthalic acid and ethylene glycol.

Further, when 1,4-butanediol as the aliphatic diol and PTMEG as the polyalkylene oxide are used together as a comonomer capable of reacting with the terephthalic acid, thermoplastic polyester elastomer (TPEE) can be obtained through a polymerization reaction of terephthalic acid, 1,4-butanediol, and PTMEG.

Further, when 1,4-butanediol as the aliphatic diol and adipic acid as the fatty acid are used together as a comonomer capable of reacting with terephthalic acid, polybutylene adipate terephthalate (PBAT), which is a type of the polyalkylene terephthalate-based copolymer, can be obtained through a polymerization reaction of terephthalic acid, 1,4-butanediol, and adipic acid.

Further, when ethylene glycol as the aliphatic diol and 1,4-cyclohexanedimethanol are used together as a comonomer capable of reacting with the terephthalic acid, a glycol-modified PET resin (glycol-modified polyethylene terephthalate, PETG), which is a type of the polyalkylene terephthalate-based copolymer, can be obtained through a polymerization reaction of terephthalic acid, ethylene glycol, and 1,4-cyclohexanedimethanol.

As a specific example, the polyalkylene terephthalate may include at least one (co)polymer selected among polybutylene terephthalate, polyethylene terephthalate, polycyclohexylene dimethylene terephthalate, polyethylene naphthalate, polybutylene naphthalate and polytrimethylene terephthalate.

Further, as a specific example, the polyalkylene terephthalate-based copolymer may include at least one (co)polymer selected among polybutylene adipate terephthalate (PBAT) and glycol-modified PET resin (glycol-modified polyethylene terephthalate, PETG).

Meanwhile, the particle size of the terephthalic acid may be 10 $\mu$m or more and 300 $\mu$m or less. The particle size of the terephthalic acid means the distance between two points where a straight line passing through the center of gravity of a weight of particles meets the boundary line of particles.

Examples of the method for measuring the particle size of the terephthalic acid are not particularly limited, and, for example, FE-SEM images and the like can be used without limitation. As for the specific methods, conditions, equipment and the like of the FE-SEM, conventionally well-known various contents can be applied without limitation. In one example of a method of measuring the particle size of the terephthalic acid, it may be measured through an FE-SEM image with HITACHI-S4800.

As described above, the particle size of terephthalic acid, which is the main synthetic target material in the present invention, is increased to 10 $\mu$m or more and 300 $\mu$m or less, which thus minimizes terephthalic acid or an impurity monomer (e.g., isophthalic acid), and increases the recovery efficiency of terephthalic acid, thereby capable of realizing excellent physical properties in the synthesis of polyethylene terephthalate or high value-added plastics (PBT, TPEE).

In particular, in the monomer composition for synthesizing recycled plastic of the one embodiment, the particle size of terephthalic acid being increased to 10 $\mu$m to 300 $\mu$m is because, through the step of dissolving the depolymerization reaction product from which the diol component has been removed in an organic solvent and then recrystallizing the product, after the step of subjecting the (co)polymer synthesized from the monomer containing terephthalic acid to a depolymerization reaction and removing the diol component as described later, the solubility of terephthalic acid or its salt is increased, and thus crystals, or impurities such as isophthalic acid interposed between crystals can be dissolved in a solvent to the maximum, and further, since the dissolved terephthalic acid has poor solubility relative to impurities, it can be easily precipitated into terephthalic acid crystals through the difference in solubility when the temperature is lowered subsequently.

In the monomer composition for synthesizing recycled plastic of the one embodiment, when the particle size of terephthalic acid is reduced to less than 10 $\mu$m, it is difficult to remove a sufficient level of impurities and secure high purity and high yield of terephthalic acid, so that the recovery process efficiency of terephthalic acid is reduced, and the physical properties of the recovered terephthalic acid and the (co)polymer synthesized therefrom may be deteriorated.

The monomer composition for synthesizing recycled plastic may further include an organic solvent having a weight ratio of less than 10 ppm based on 100% by weight of the monomer composition for synthesizing recycled plastic. The organic solvent corresponds to the organic solvent used in the recrystallization step described later during the recovery step of the monomer composition for synthesizing recycled plastic.

Specific examples of the organic solvent used in the recrystallization step are not particularly limited, and various organic solvents widely used in the existing recrystallization technology field of terephthalic acid can be applied without limitation. However, as an example of the organic solvent, N-methyl-2-pyrrolidone or dimethylacetamide can be used.

In particular, in the case of the N-methyl-2-pyrrolidone or dimethylacetamide solvent, terephthalic acid and other impurities can be sufficiently dissolved even at a relatively low temperature, and then when the solution is cooled, the difference in solubility between terephthalic acid and other impurities at the cooling temperature is large, so that only terephthalic acid can be efficiently recrystallized and separated.

That is, this means that recovery is performed from the (co)polymer synthesized from the monomer containing terephthalic acid in order to obtain the monomer composition for synthesizing recycled plastic of the one embodiment, and as a result, terephthalic acid is also obtained together. Therefore, apart from the recovery from the (co)polymer synthesized from the monomer containing terephthalic acid in order to prepare the monomer composition for synthesizing recycled plastic of the one embodiment, the case where new organic solvent is added from the outside is not included in the scope of organic solvent of the present invention.

Specifically, "recovered from the (co)polymer synthesized from the monomer containing terephthalic acid" means that it was obtained by a depolymerization reaction of a (co)polymer synthesized from a monomer including terephthalic acid. The depolymerization reaction can be carried out under acidic, neutral or basic conditions, and in particular, the depolymerization reaction can be carried out under basic (alkaline) conditions. More specifically, the organic solvent can be derived from a recrystallization solvent used for recrystallization for high-purity purification of terephthalic acid after depolymerization reaction of the (co)polymer synthesized from the monomer containing terephthalic acid.

The weight ratio of the organic solvent which is an impurity other than terephthalic acid, which is the main synthesis target material in the present invention, is extremely reduced to less than 10 ppm based on 100% by weight of the monomer composition for synthesizing recycled plastic, thereby capable of realizing excellent physical properties in the synthesis of polyethylene terephthalate or high value-added plastics (PBT, TPEE).

Examples of a method for measuring the weight ratio of the organic solvent are not particularly limited, and, for example, $^1$H NMR, ICP-MS analysis, HPLC analysis, and the like can be used without limitation. As for the specific methods, conditions, devices and the like of the NMR, ICP-MS and HPLC, conventionally well-known various contents can be applied without limitation.

In an example of a method for measuring the molar ratio of the organic solvent, 0.5 mg of the monomer composition for synthesizing recycled plastic is collected as a sample under the conditions of normal pressure, 20°C or more and 30°C or less, inorganic matter analysis is carried out through ICP-OES equipment, and the weight ratio (% by weight) of the organic solvent contained therein is measured based on 100% by weight of the sample.

In particular, in the monomer composition for synthesizing recycled plastic of the one embodiment, the ratio of the organic solvent, which is an impurity monomer other than terephthalic acid, being extremely reduced to less than 10 ppm based on 100% by weight of the monomer composition for synthesizing recycled plastic is because the recrystallization organic solvent is vaporized and removed at a temperature exceeding the boiling point of the recrystallization organic solvent through the step of washing the result of the recrystallization step with a solvent at a temperature of 200°C or more and 300°C or less as described below.

In the monomer composition for synthesizing recycled plastic of the one embodiment, when the weight ratio of the organic solvent, which is an impurity monomer other than terephthalic acid, is increased to more than 10 ppm based on 100% by weight of the monomer composition for synthesizing recycled plastic, the physical properties of the recovered terephthalic acid and the (co)polymer synthesized therefrom may be deteriorated due to the recrystallization organic solvent remaining excessively in terephthalic acid.

The monomer composition for synthesizing recycled plastic of the one embodiment may further include some small amounts of other additives and solvents, and the types of specific additives and solvents are not particularly limited, and various materials widely used in the terephthalic acid recovery step by depolymerization of a (co)polymer synthesized from a monomer containing terephthalic acid can be applied without limitation.

The monomer composition for synthesizing recycled plastic of the one embodiment can be obtained by a method for preparing a monomer composition for synthesizing recycled plastic, which will be described later. That is, the monomer composition for synthesizing recycled plastics of the one embodiment corresponds to a product obtained through various filtration, purification, washing, and drying steps, in order to secure only terephthalic acid in high purity, which is the main synthetic target material in the present invention, after depolymerization of a (co)polymer synthesized from a monomer containing terephthalic acid.

The monomer composition for synthesizing recycled plastics of the one embodiment can be applied as a raw material for the preparation of monomers used in the synthesis of various recycled plastics (e.g., polyethylene terephthalate (PET), polybutylene terephthalate (PBT), polybutylene adipate terephthalate (PBAT), glycol-modified PET resin (glycol-modified polyethylene terephthalate, PETG), thermoplastic polyester elastomer (TPEE)), which will be described later, or can be used as a raw material for the preparation of other additives (e.g., dioctylterephthalate plasticizer) used in the processing of plastics (e.g., polyvinyl chloride (PVC)).

2. Method for preparing a monomer composition for synthesizing recycled plastic

According to another embodiment of the invention, there can be provided a method for preparing a monomer composition for synthesizing recycled plastic, comprising the steps of: subjecting a (co)polymer synthesized from a monomer containing terephthalic acid to a depolymerization reaction and removing a diol component; dissolving the depolymerization reaction product from which the diol component has been removed in an organic solvent and then recrystallizing the product; and washing the result of the recrystallization step with a solvent at a temperature of 200°C or more and 300°C or less.

The present inventors confirmed through experiments that similarly to the method for preparing the monomer composition for synthesizing recycled plastic of the other embodiments, since a recrystallization step of dissolving the reaction product in an organic solvent and then recrystallizing it; and a washing process of washing the result of the recrystallization step with a solvent at a temperature of 200°C or more and 300°C or less are applied in the step of recovering terephthalic acid from a (co)polymer synthesized from a monomer containing terephthalic acid, the ratio of isophthalic acid, which is other monomers and not terephthalic acid, which is the main synthetic target material in the present invention, and the recrystallization organic solvent is extremely reduced, thereby capable of realizing excellent physical properties in the synthesis of polyethylene terephthalate or high value-added plastics (PBT, TPEE) using this, and completed the present invention.

In particular, terephthalic acid recovered from a (co)polymer synthesized from a monomer containing terephthalic acid obtained by conventional methods usually contains 1 to 2% isophthalic acid as an impurity, whereas in the present invention, isophthalic acid or other impurities could be almost completely removed by a high-temperature recrystallization step which is characteristic in the method for preparing the monomer composition for synthesizing recycled plastics, which will be described later.

This is because, through the step of dissolving the depolymerization reaction product from which the diol component has been removed in an organic solvent and then recrystallizing the product, the solubility of terephthalic acid contained in the depolymerization reaction product is increased and thus, crystals, or impurities such as isophthalic acid interposed between crystals can be dissolved in a solvent to the maximum, and further, since the dissolved terephthalic acid has poor solubility relative to impurities, it can be easily precipitated into terephthalic acid crystals through the difference in solubility when the temperature is lowered subsequently.

Further, through the step of washing with a solvent at a temperature of 200°C or more and 300°C or less, the solubility of terephthalic acid or its salt is increased and thus, crystals, or impurities such as isophthalic acid interposed between crystals can be dissolved in the solvent to the maximum, and further, the temperature can be lowered using the difference in solubility between the dissolved terephthalic acid and impurities to recover terephthalic acid again.

Specifically, the method for preparing the monomer composition for synthesizing recycled plastic of the other embodiment may include a step of subjecting the (co)polymer synthesized from the monomer containing terephthalic acid to a depolymerization reaction and removing the diol component.

The (co)polymer synthesized from the monomer containing terephthalic acid can be applied regardless of various forms and types, such as a (co)polymer synthesized from a monomer containing novel terephthalic acid produced through synthesis, a (co)polymer synthesized from a monomer containing recycled terephthalic acid produced through a recycling step; or a (co)polymer waste synthesized from monomers containing terephthalic acid, and the like.

However, if necessary, before proceeding with the depolymerization reaction of the (co)polymer synthesized from the monomer containing terephthalic acid, a pretreatment step of the (co)polymer synthesized from the monomer containing terephthalic acid can be carried out, thereby increasing the efficiency of the step of recovering terephthalic acid from the (co)polymer synthesized from the monomer containing terephthalic acid. Examples of the pretreatment step may include washing, drying, grinding, glycol decomposition, and the like. The specific method of each pretreatment process is not limited, and various methods widely used in the recovery step of terephthalic acid by depolymerization of a (co)polymer synthesized from a monomer containing terephthalic acid can be applied without limitation.

In the depolymerization reaction of the (co)polymer synthesized from the monomer containing terephthalic acid, the depolymerization reaction can be carried out under acidic, neutral, or basic conditions. For example, when the depolymerization reaction proceeds under basic (alkaline) conditions, a terephthalic acid salt called $Na_2$-TPA and ethylene glycol are primarily produced from the polyethylene terephthalate, and $Na_2$-TPA is converted to TPA through secondary strong acid neutralization, thereby capable of recovering terephthalic acid.

More specifically, the depolymerization reaction of the (co)polymer synthesized from the monomer containing terephthalic acid can be carried out in the presence of water, an alkylene glycol, or an alcohol solvent. Specific examples of the alkylene glycol solvent include ethylene glycol, and specific examples of the alcohol solvent include ethanol.

Further, the depolymerization reaction of the (co)polymer synthesized from the monomer containing terephthalic acid can be carried out under basic conditions. The type of the base is not particularly limited, and examples thereof include sodium hydroxide (NaOH).

The depolymerization reaction of the (co)polymer synthesized from the monomer containing terephthalic acid can be carried out by reacting a base in an amount of 2.5 mol or less, 1.5 mol or more and 2.5 mol or less, or 1.5 mol or more and 2.3 mol or less relative to 1 mol of a (co)polymer synthesized from a monomer containing terephthalic acid.

In the depolymerization reaction of the (co)polymer synthesized from the monomer containing terephthalic acid, when the base is reacted in an amount or more than 2.5 mol relative to 1 mol of the (co)polymer synthesized from the monomer containing terephthalic acid, isophthalic acid and sodium (Na), which are impurities other than terephthalic acid, which are the main synthetic target materials, are increased due to the influence of the decrease in the solubility of isophthalate and the increase in the amount of alkaline salt generated, which makes it difficult to sufficiently remove it even by a washing step described later.

Further, the temperature at which the depolymerization reaction of the (co)polymer synthesized from the monomer containing terephthalic acid proceeds is not particularly limited, but for example, the reaction can proceed at a temperature of 25°C or more and 200°C or less, or 130°C or more and 180°C or less. In addition, the time required for the depolymerization reaction of the (co)polymer synthesized from the monomer containing terephthalic acid may be 0 minutes or more and 3 hours or less.

Meanwhile, after the (co)polymer synthesized from the monomer containing terephthalic acid is subjected to a depolymerization reaction, the diol component can be removed. For example, the polyethylene terephthalate (PET) alkaline decomposition product includes ethylene glycol and terephthalate.

Since the main recovery target material of the present invention is terephthalic acid, other by-products can be removed through filtration. The filtered by-products can be recycled without a separation and purification process in the depolymerization reaction of the (co)polymer synthesized from the monomer containing terephthalic acid, or if necessary, can be recycled through separation and purification using conventional distillation, extraction, and adsorption methods.

As a specific example, after the product of the depolymerization reaction of polyethylene terephthalate is cooled to 50°C or less, ethylene glycol can be removed through vacuum filtration to obtain terephthalate.

Moreover, since the main recovery target material of the present invention is terephthalic acid, in the case of terephthalic acid, it can be converted to terephthalic acid through a neutralization process with an additional strong acid as described below.

Meanwhile, the method for preparing the monomer composition for synthesizing recycled plastic of another embodiment may include a step of dissolving the depolymerization reaction product from which the diol component has been removed in an organic solvent and then recrystallizing the product.

As described above, in the depolymerization reaction product from which the diol component has been removed, ethylene glycol is removed, and terephthalate, or terephthalic acid obtained by its neutralization can be contained. However, since various impurities remain during the recovery step of obtaining terephthalic acid, recrystallization can proceed in order to sufficiently remove this and secure high-purity terephthalic acid.

Specifically, the recrystallization step may include a step of dissolving the depolymerization reaction product from which the diol component has been removed in an organic solvent and then recrystallizing it. Through the step of dissolving the depolymerization reaction product from which the diol component has been removed in an organic solvent and then recrystallizing it, the solubility of terephthalic acid or a salt thereof contained in the depolymerization reaction product is increased and thus, crystals, or impurities such as isophthalic acid interposed between crystals can be dissolved with a solvent to the maximum, and further, since the dissolved terephthalic acid has poor solubility relative to impurities, it can be easily precipitated into terephthalic acid crystals through the difference in solubility when the temperature is lowered subsequently.

Specific examples of the organic solvent used in the recrystallization step are not particularly limited, and various organic solvents widely used in the existing recrystallization technology field of terephthalic acid can be applied without limitation. However, in an illustrative example of the organic solvent, N-methyl-2-pyrrolidone or dimethylacetamide can be used.

In the case of the N-methyl-2-pyrrolidone or dimethylacetamide solvent, terephthalic acid and other impurities can be sufficiently dissolved even at a relatively low temperature, and then, when the solution is cooled, the difference in solubility between terephthalic acid and other impurities at the cooling temperature is large, so that only terephthalic acid can be efficiently recrystallized and separated.

4 Parts by weight or more and 10 parts by weight or less of the organic solvent may be used based on 1 part by weight of the depolymerization reaction product from which the diol component has been removed. When the organic solvent is used in an amount of less than 4 parts by weight based on 1 part by weight of the depolymerization reaction product from which the diol component has been removed, the temperature for dissolving the terephthalic acid contained in the depolymerization reaction product from which the diol component has been removed becomes too high, which leads to decrease in process efficiency, and it is not easy to remove impurities through recrystallization. On the other hand, when the organic solvent is used in an amount of more than 10 parts by weight based on 1 part by weight of the depolymerization reaction product from which the diol component has been removed, the solubility of terephthalic acid contained in the depolymerization reaction product from which the diol component has been removed becomes excessively high, so that the yield of terephthalic acid recovered after recrystallization decreases, and the process efficiency can be decreased due to the use of large amounts of solvent.

More specifically, the recrystallization step may include a step of dissolving the depolymerization reaction product from which the diol component has been removed in an organic solvent at a temperature of 50°C or more and 130°C or less, or 50°C or more and 100°C or less, or 50°C or more and 70°C or less; and a step of cooling the dissolved solution to a temperature of 10°C or less, or 0°C or more and 10°C or less, or 5°C or more and 10°C or less.

Despite the high temperature range and the cooling temperature range where recrystallization proceeds are relatively mild conditions, there is an advantage in that impurities can be removed at a high level and the terephthalic acid recovery efficiency can be increased. The temperature condition means the temperature inside the container in which recrystallization by the solvent proceeds, and various heating and cooling devices can be applied without limitation in order to maintain high and low temperatures outside the normal temperature.

Specific examples of the dissolution, cooling conditions, apparatus, and method of the recrystallization step are not particularly limited, and various methods widely used in the existing recrystallization technology field of terephthalic acid are applicable without limitation.

Meanwhile, the method for preparing the monomer composition for synthesizing recycled plastic of the other embodiment may include a step of washing the result of the recrystallization step with a solvent at a temperature of 200°C or more and 300°C or less.

Through the step of washing the result of the recrystallization step with a solvent at a temperature of 200°C or more and 300°C or less, the solubility of terephthalic acid or a salt thereof can be increased to thereby dissolve impurities such as crystals or isophthalic acid interposed between crystals with a solvent to the maximum, and the temperature can be lowered using the difference in solubility between the dissolved terephthalic acid and the impurities to recover the terephthalic acid again.

The result of the recrystallization step may contain terephthalic acid, a salt thereof, or a mixture thereof. However, since various impurities remain during the recovery process of obtaining terephthalic acid, washing can proceed in order to sufficiently remove this and secure high-purity terephthalic acid.

Specifically, the washing step may include a step of washing the result of the recrystallization step with a solvent at a temperature of 200°C or more and 300°C or less, or 205°C or more and 300°C or less, or 200°C or more and 250°C or less, or 205°C or more and 250°C or less. The temperature condition refers to the temperature inside the washing container in which washing with a solvent is carried out. Various heating devices can be applied without limitation in order to maintain a high temperature outside the normal temperature.

When the temperature at which the result of the recrystallization step is washed is reduced to less than 200°C, there is a limit in that the solubility of terephthalic acid is hard to be sufficiently increased, which makes it difficult to dissolve crystals, or impurities such as isophthalic acid interposed between crystals with a solvent to the maximum. Further, it is difficult to sufficiently remove the recrystallization organic solvent while forming a temperature lower than the boiling point of the solvent used for recrystallization. On the other hand, if the temperature for washing the result of the recrystallization step is excessively increased to greater than 300°C, harsh conditions are created to maintain extreme temperature conditions, which can reduce the process efficiency.

The solvent used in the washing step may be used in a weight ratio of 5 parts by weight or more and 10 parts by weight or less based on 1 part by weight of the result of the recrystallization step. When the washing solvent is used in an amount of less than 5 parts by weight based on 1 part by weight of the result of the recrystallization step, there is a limit in that the solubility of terephthalic acid is hard to be sufficiently increased, which makes it difficult to dissolve crystals, or impurities such as isophthalic acid interposed between crystals with a solvent at the maximum. On the other hand, when the washing solvent is used in an amount of greater than 10 parts by weight based on 1 part by weight of the result of the recrystallization step, the process efficiency can be reduced due to the use of large amounts of solvent.

The step of washing the result of the recrystallization step with a solvent at a temperature of 200°C or more and 300°C or less can proceed repeatedly at least once or more.

Further, if necessary, after proceeding the step of washing the result of the recrystallization step with a solvent at a temperature of 200 °C or more and 300 °C or less, a step of removing the residual solvent through filtration may be further performed.

Meanwhile, the method for preparing the monomer composition for synthesizing recycled plastic of the other embodiment may further include a cooling step after the step of washing the depolymerization reaction product from which the diol component has been removed. Crystallized terephthalic acid can be obtained by filtration or the like through the difference in solubility between terephthalic acid and impurities by cooling, and the impurities can be removed in a state of being dissolved in a solvent. The specific cooling conditions are not particularly limited, but for example, cooling can proceed at a temperature of 100°C or less, or 50°C or more and 100°C or less. As for the specific drying device and method used during the cooling, various known cooling techniques can be applied without limitation.

Meanwhile, the method for preparing the monomer composition for synthesizing recycled plastic of the other embodiment may further include a step of subjecting the depolymerization product from which the diol component has been removed to a neutralization reaction with an acid, before the step of washing the depolymerization reaction product from which the diol component has been removed.

For example, polyethylene terephthalate (PET) alkaline decomposition products include ethylene glycol and terephthalate, but since the main recovery target material of the present invention is terephthalic acid, in the case of the terephthalate obtained by the alkaline decomposition, it can be converted to terephthalic acid through a further neutralization step with a strong acid. That is, when the depolymerization reaction of the (co)polymer synthesized from the monomer containing terephthalic acid is alkaline decomposition, it can undergo a neutralization reaction step with an acid.

The acid used in the neutralization reaction may be a strong acid, and examples thereof include hydrochloric acid (HCl). Due to the neutralization reaction by the strong acid, the pH may be 4 or less, or 2 or less at the time of completion of the neutralization reaction. The temperature during the neutralization reaction can be adjusted to 25°C or more and 100 °C or less.

Further, if necessary, after proceeding the step of a neutralization reaction step by acid of the depolymerization reaction product from which the diol component has been removed, a step of removing residual impurities through filtration can be further performed.

Meanwhile, the method for preparing a monomer composition for synthesizing recycled plastic may further include a step of purifying the depolymerization reaction product from which the diol component has been removed, after the step of subjecting a (co)polymer synthesized from a monomer containing terephthalic acid to a depolymerization reaction and removing a diol component.

Residual impurities can be removed through the purification, and specific purification conditions are not particularly limited. As for specific purification devices and methods, various purification techniques well-known conventionally can be applied without limitation.

In one specific example, the purification step of the depolymerization reaction product from which the diol component has been removed may include a step of dissolving and filtering the depolymerization reaction product from which the diol component has been removed; and an adsorption step through an adsorbent.

In the step of dissolving and filtering the depolymerization reaction product from which the diol component has been removed, water may be used as a solvent for dissolving the depolymerization reaction product from which the diol component has been removed, and the dissolution temperature may be 25°C or more and 100°C or less. Thereby, it is possible to remove the (co)polymer synthesized from the monomer containing residual terephthalic acid that did not react in the depolymerization reaction.

In the adsorption step through the adsorbent, examples of the adsorbent may be activated carbon, charcoal, celite, or a mixture thereof.

In the purification step of the depolymerization reaction product from which the diol component has been removed, if necessary, an extraction step, a washing step, a precipitation step, a recrystallization step, a drying step, and the like can be further applied without limitation.

When the purification step of the depolymerization reaction product from which the diol component has been removed is performed, after the purification of the depolymerization reaction product from which the diol component has been removed, a step of dissolving the depolymerization reaction product from which the diol component has been removed in an organic solvent and then recrystallizing the product can be performed.

Further, as described above, in the case where the depolymerization reaction is an alkaline decomposition, if the purification step of the depolymerization reaction product from which the diol component has been removed is performed, after the purification step of the depolymerization reaction product from which the diol component has been removed, a step of subjecting the depolymerization reaction product from which the diol component has been removed to a neutralization reaction with an acid, and then a step of dissolving the depolymerization reaction product from which the diol component has been removed in an organic solvent and then recrystallizing the product can be performed.

That is, the method for preparing the monomer composition for synthesizing recycled plastic of the other embodiment can proceed in the order of a step of subjecting a (co)polymer synthesized from a monomer containing terephthalic acid to a depolymerization reaction and removing a diol component, a step of purifying the depolymerization reaction product from which the diol component has been removed, a step of subjecting the depolymerization reaction product from which the diol component has been removed to a neutralization reaction with an acid, and a step of dissolving the depolymerization reaction product from which the diol component has been removed in an organic solvent and then recrystallizing it.

On the other hand, the method for preparing the monomer composition for synthesizing recycled plastic of the other embodiment may further include a drying step, after the step of washing the depolymerization reaction product from which the diol component has been removed. The residual solvent can be removed by the drying, and the specific drying conditions are not particularly limited, but for example, the drying can proceed at a temperature of 100°C or more and 150°C or less. As for specific drying devices and methods used during the drying, various known drying techniques can be applied without limitation.

3. Recycled Plastic

According to another embodiment of the invention, there can be provided a recycled plastic comprising a reaction product of the monomer composition for synthesizing recycled plastic of the one embodiment and a comonomer. The details of the monomer composition for synthesizing recycled plastic includes all the contents described above in the one embodiment.

Examples corresponding to the recycled plastic are not particularly limited, and various plastics synthesized using terephthalic acid as a monomer can be applied without limitation, and a more specific example may be a (co)polymer synthesized from a monomer containing terephthalic acid.

In the (co)polymer synthesized from the monomer containing terephthalic acid, the (co)polymer includes both a polymer or a copolymer, and collectively refers to a reaction product obtained through (co)polymerization of monomers. The (co)polymer may include all low molecular weight compounds, oligomers, and polymers depending on the molecular weight range.

The (co)polymer synthesized from the monomer containing terephthalic acid may include at least one (co)polymer selected from the group consisting of polyalkylene terephthalate, polyalkylene terephthalate-based copolymer, and thermoplastic polyester elastomer. That is, the (co)polymer synthesized from the monomer containing terephthalic acid may include one type of polyalkylene terephthalate, one type of polyalkylene terephthalate-based copolymer, one type of thermoplastic polyester elastomer, or a mixture of two or more thereof

The polyalkylene terephthalate-based copolymer means a copolymer obtained by further reacting an additional comonomer based on alkylene glycol and terephthalic acid which are monomers for synthesizing polyalkylene terephthalate.

Examples of the comonomer capable of reacting with high-purity terephthalic acid contained in the monomer composition for synthesizing recycled plastic of the one embodiment are also not particularly limited, and specific examples thereof include aliphatic diols, polyalkylene oxides, fatty acids, fatty acid derivatives, or combinations thereof.

As the aliphatic diol, for example, a diol having a number average molecular weight (Mn) of 300 g/mol or less, that is, at least one selected among ethylene glycol, propylene glycol, 1,2-propanediol, 1,3-propanediol, 1,4-butanediol (1,4-BG), 1,5-pentanediol, 1,6-hexanediol, and 1,4-cyclohexanedimethanol (1,4-CHDM) can be used, and as a specific example, 1,4-butanediol, ethylene glycol, 1,4-cyclohexanedimethanol, or a mixture thereof can be used.

The polyalkylene oxide is a unit constituting a soft fraction, and may include an aliphatic polyether as a constituent component. As an example, at least one selected among polyoxyethylene glycol, polypropylene glycol, poly(tetramethylene ether)glycol (PTMEG), polyoxyhexamethylene glycol, a copolymer of ethylene oxide and propylene oxide, an ethylene oxide addition polymer of polypropylene oxide glycol, a copolymer of ethylene oxide and tetrahydrofuran can be used, and as a specific example, PTMEG can be used. Particularly, PTMEG having a number average molecular weight (Mn) of 600 g/mol to 3,000 g/mol, 1,000 g/mol to 2,500 g/mol, or 1,500 g/mol to 2,200 g/mol can be used.

The fatty acid may be at least one type of aliphatic carboxylic acid compounds excluding terephthalic acid as an example, and adipic acid can be used as a specific example. The fatty acid derivative is a compound derived from the above-mentioned fatty acid, and as an example, at least one selected among fatty acid ester, fatty acid chloride, fatty acid anhydride, and fatty acid amide can be used, and as a specific example, adipic acid ester can be used.

In a more specific example, when 1,4-butanediol, which is the aliphatic diol, is used as a comonomer capable of reacting with the terephthalic acid, polybutylene terephthalate (PBT), which is a type of polyalkylene terephthalate, can be obtained by polymerization reaction of terephthalic acid and 1,4-butanediol.

Further, when ethylene glycol, which is an aliphatic diol, is used as a comonomer capable of reacting with high-purity terephthalic acid contained in the monomer composition for synthesizing recycled plastic of the one embodiment, polyethylene terephthalate (PET), which is a type of the polyalkylene terephthalate, can be obtained through a polymerization reaction of terephthalic acid and ethylene glycol.

Further, when 1,4-butanediolas the aliphatic diol and PTMEG as the polyalkylene oxide are used together as a comonomer capable of reacting with high-purity terephthalic acid contained in the monomer composition for synthesizing recycled plastic of the one embodiment, a thermoplastic polyester elastomer (TPEE) can be obtained through a polymerization reaction of terephthalic acid, 1,4-butanediol, and PTMEG.

Further, when 1,4-butanediol as the aliphatic diol and adipic acid as the fatty acid are used together as a comonomer capable of reacting with high-purity terephthalic acid contained in the monomer composition for synthesizing recycled plastic of the one embodiment, polybutylene adipate terephthalate (PBAT), which is a type of the polyalkylene terephthalate-based copolymer, may be obtained through a polymerization reaction of terephthalic acid, 1,4-butanediol, and adipic acid.

Further, when the aliphatic diol ethylene glycol and 1,4-cyclohexanedimethanol are used together as a comonomer capable of reacting with terephthalic acid of high purity contained in the monomer composition for synthesizing recycled plastic of the on embodiment, a glycol-modified PET resin (glycol-modified polyethylene terephthalate, PETG), which is a kind of the polyalkylene terephthalate-based copolymer, can be obtained through a polymerization reaction of terephthalic acid, ethylene glycol, and 1,4-cyclohexanedimethanol.

As a specific example, the polyalkylene terephthalate may include at least one (co)polymer selected among polybutylene terephthalate, polyethylene terephthalate, polycyclohexylene dimethylene terephthalate, polyethylene naphthalate, polybutylene naphthalate and polytrimethylene terephthalate.

Further, as a specific example, the polyalkylene terephthalate-based copolymer may include at least one (co)polymer selected from polybutylene adipate terephthalate (PBAT) and glycol-modified PET resin (glycol-modified polyethylene terephthalate, PETG).

Examples of the reaction method of the monomer composition for synthesizing recycled plastic and the comonomer are not particularly limited, and various known methods can be applied without limitation. However, specific examples of the reaction of the monomer composition for synthesizing recycled plastic and the comonomer may include melt-polycondensation and solid-state polymerization.

As a more specific example, when preparing a thermoplastic polyester elastomer (TPEE) from the recycled plastic, aromatic dicarboxylic acid, aliphatic diol and polyalkylene oxide is subjected to an esterification reaction in the presence of a titanium butoxide (TBT) catalyst at 180°C or more and 250°C or less for 30 minutes or more and 210 minutes or less to produce BHBT (bis(4-hydroxy)butyl terephthalate) oligomer, and then the TBT catalyst is re-charged, and the melt-polycondensation reaction may be carried out at 200°C or more and 270°C or less for 20 minutes or more and 240 minutes or less while gradually reducing the pressure from 760 torr to 0.3 torr. After the completion of the melt-polycondensation reaction, it may be discharged into the reactor under nitrogen pressure to pelletize the strands to form a pellet.

Then, solid-phase polymerization of the pellets in a solid-phase polymerization reactor or a rotatable vacuum dryer in a temperature range of 140°C or more and 200°C or less for 10 hours or more and 24 hours or less can be carried out under an inert air flow such as nitrogen under high vacuum.

Further, when producing polyalkylene terephthalate from the recycled plastic, an aromatic dicarboxylic acid and an aliphatic diol having a number average molecular weight (Mn) of 300 g/mol or less may be melt-polymerized and then solid-phase polymerized.

For the polyalkylene terephthalate resin, the low molecular weight pellets obtained by melt polymerization is placed in a solid-phase polymerization reactor, and reacted under high vacuum and inert conditions as presented in the solid-phase polymerization of the thermoplastic polyester elastomer (TPEE) described above, thereby obtaining a high molecular weight resin.

Due to the reaction of the monomer composition for synthesizing recycled plastic and the comonomer, the specific method for preparing the polybutylene terephthalate, polyethylene terephthalate, polycyclohexylene dimethylene terephthalate, polyethylene naphthalate, polybutylene naphthalate, polytrimethylene terephthalate, or polybutylene adipate terephthalate is not particularly limited, and various processes widely known in in the field of the existing recycled plastic synthesis can be applied without limitation.

The recycled plastic may have a melt flow index of 15 g/10 min or more and 27 g/10 min or less as measured by ASTM D1238. When the melt flow index of the recycled plastic increases to more than 27 g/10 min, molding of the recycled plastic is not easy.

However, in an example of the method for measuring the melt flow index of the recycled plastic, it can be measured at 230°C to 250°C and a load of 2.16 kg for 4 minutes according to ASTM D1238.

More specifically, for example, in the case of a thermoplastic polyester elastomer (TPEE), the melt flow index can be measured at 230°C and a load of 2.16 kg for 4 minutes according to ASTM D1238. Further, in the case of the polyalkylene terephthalate, the melt flow index can be measured at 250°C and a load of 2.16 kg for 4 minutes according to ASTM D1238.

The melt flow index characteristics of the recycled plastic may vary depending on the weight ratio of the monomer composition and comonomer for synthesizing the recycled plastic of the one embodiment, and the weight ratio of the monomer composition for synthesizing recycled plastic and the comonomer of the one embodiment is not particularly limited. For example, based on the thermoplastic polyester elastomer (TPEE), it may be synthesized at a weight ratio of TPA/(TPA+PTMG)=61.5.

4. Molded Product

According to another embodiment of the invention, a molded article comprising the recycled plastic of the other embodiment can be provided. The details of the recycled plastic includes all the contents described above in the other embodiments.

The molded article can be obtained by applying the recycled plastic to various known plastic molding methods without limitation. As an example of the molding method, injection molding, foam injection molding, blow molding, or extrusion molding may be mentioned.

Examples of the molded article are not particularly limited, and can be applied to various molded articles using plastic without limitation. Examples of the molded article include automobiles, electrical and electronic products, communication products, and daily necessities.

5. Plasticizer Composition

According to another embodiment of the invention, there can be provided a plasticizer composition comprising a reaction product of the monomer composition for synthesizing recycled plastic of the one embodiment and an alcohol. The details of the monomer composition for synthesizing the recycled plastic includes all the contents described above in the one embodiment.

Generally, a plasticizer is obtained by appropriately adding a resin such as polyvinyl chloride (PVC) and various additives such as a filler, a stabilizer, a pigment, and an antifogging agent to impart various processed physical properties, and is used as materials for a variety of products, ranging from wires, pipes, flooring, wallpaper, sheets, artificial leather, tarpaulin, tapes, and food packaging materials by processing methods such as extrusion molding, injection molding, and calendaring.

Typically, for the plasticizer, the alcohol reacts with polycarboxylic acid such as phthalic acid and adipic acid to form the corresponding ester. In addition, in consideration of domestic and international regulations on phthalate-based plasticizers that are harmful to the human body, research on plasticizer compositions that can replace phthalate-based plasticizers such as terephthalate-based, trimellitate-based, and other polymer-based plasticizers is being continued.

The reaction product of the monomer composition for synthesizing recycled plastic of the one embodiment and alcohol may include a terephthalate-based compound. Specifically, a terephthalate-based compound can be obtained through a direct esterification reaction between terephthalic acid and alcohol contained in the monomer composition for synthesizing recycled plastic of the one embodiment.

The direct esterification reaction may comprise the steps of adding terephthalic acid to alcohol, then adding a catalyst and reacting the mixture under a nitrogen atmosphere; removing unreacted alcohol and neutralizing unreacted acid; and dehydrating and filtrating the result by distillation under reduced pressure.

Examples of the terephthalate-based compound are not particularly limited, but examples thereof include dioctyl terephthalate (DOTP), diisononyl terephthalate (DINTP), diisodecyl terephthalate (DIDTP), or di(2-propylheptyl)terephthalate (DPHTP) and the like.

The terephthalate-based compound can be prepared through a direct esterification reaction in which any one alcohol selected from the group consisting of octanol, isononyl alcohol, isodecyl alcohol and 2-propylheptyl alcohol, and terephthalic acid are reacted.

The plasticizer composition can be applied to the manufacture of electric wires, flooring materials, automobile interior materials, films, sheets, wallpaper or tubes.

[Advantageous Effects]

According to the present invention, a monomer composition for synthesizing recycled plastic that is capable of remarkably reducing the content of isophthalic acid, which is an impurity, and thus increasing the purity of terephthalic acid, when recovering terephthalic acid through a depolymerization reaction of a (co)polymer synthesized from a monomer containing terephthalic acid, and also of securing terephthalic acid having increased particle size through recrystallization, a method for preparing the same, and a recycled plastic, molded product, and plasticizer composition using the same can be provided.

[DETAILED DESCRIPTION OF THE EMBODIMENTS]

Hereinafter, the present invention will be explained in detail with reference to the following examples. However, these examples are for illustrative purposes only, and the scope of the present invention is not limited thereto.

<EXAMPLE>

Example 1

(1) Preparation of recycled terephthalic acid monomer composition

300 g (1.56 mol) of polyethylene terephthalate (PET) bottle scrap, 1939.69 g (31.25 mol) of ethylene glycol (EG), and 128.13 g (3.20 mol) of sodium hydroxide (NaOH) were placed in a 3L SUS reactor, and then stirred at 180°C for 2 hours in a closed system to proceed a PET depolymerization reaction. The product of the depolymerization reaction was cooled to 50°C or less, and then, filtered under vacuum to obtain sodium terephthalate ($Na_2$-TPA).
The filtrate containing sodium terephthalate ($Na_2$-TPA) was completely dissolved in 3000 g of water, and then filtered under vacuum again to remove unreacted PET.
Then, 15 g of charcoal was added to the filtrate, stirred for 1 hour, and then filtered to remove a charcoal. Then, about 500 ~ 550 ml of 6N HCl was added and neutralized at 20 ~ 30°C, and the slurry lowered to pH 2 was filtered under vacuum again to obtain terephthalic acid (TPA).
N-methyl-2-pyrrolidone (NMP) was added in an amount of 10 times relative to the amount of the obtained terephthalic acid (TPA), heated to 60°C and completely dissolved at 60°C. Then, the TPA/NMP solution was cooled up to 10°C, and recrystallized to recover terephthalic acid (TPA) crystals.
The recovered terephthalic acid (TPA) crystals, and water in an amount of 10 times relative to the amount of terephthalic acid (TPA) were placed in a 3L SUS highpressure vessel, heated to 205°C, washed at 205°C, slowly cooled to 100°C or less, atmospheric pressure, and then filtered under vacuum.
After performing the washing step, it was dried in a convection oven at 100°C for 12 hours to prepare a recycled terephthalic acid monomer composition in which recycled terephthalic acid (TPA) was recovered.

(2) Preparation of recycled plastic

200 g of the recycled terephthalic acid monomer composition obtained in Example 1(1), 200 g of 1,4-butylene glycol, and 125 g of poly(tetramethylene) glycol (PTMEG) having a number average molecular weight of 1,000~2,000 g/mol was placed in an ester interchange reactor, and then 0.1 wt% of TBT catalyst was added thereto. The mixture was reacted for 120-180 minutes while maintaining 200~240°C, and the reaction was terminated when the reaction rate (the value obtained by converting the amount of water, which is the reaction effluent, into the reaction rate) was 90% or more to obtain an oligomer.
Then, the prepared oligomer was transferred to a polycondensation reactor, and 0.1wt% of TBT catalyst, 0.14~0.15wt% of hindered phenolic antioxidant, and 0.15~0.2wt% of aromatic amine antioxidant or sulfur-based stabilizer were added thereto. The pressure was reduced from 760 torr to 0.3 torr for 30 minutes while maintaining 230~250°C, a melt polycondensation reaction was carried out, and then melt polycondensation was carried out under high vacuum conditions of 0.3 torr or less until the torque applied to the stirrer reached the desired torque. After completion of the reaction, it was discharged using nitrogen pressure, stranded, cooled, and then pelletized to prepare a thermoplastic polyester elastomer (TPEE) resin.

Example 2

A recycled terephthalic acid monomer composition and a recycled plastic were prepared in the same manner as in Example 1, except that the amount of the recrystallization solvent NMP used in Example 1(1) was changed to 5 times relative to that of the obtained terephthalic acid (TPA) as shown in Table 1 below.

Example 3

A recycled terephthalic acid monomer composition and a recycled plastic were prepared in the same manner as in Example 1, except that the amount of recrystallization solvent NMP used in Example 1(1) was changed to 4 times relative to that of the obtained terephthalic acid (TPA) as shown in Table 1 below.

Example 4

A recycled terephthalic acid monomer composition and a recycled plastic were prepared in the same manner as in Example 1, except that the amount of recrystallization solvent NMP used in Example 1(1) was changed to 4 times relative to that of the obtained terephthalic acid (TPA), and the amount of washing water used was changed to 5 times relative to that of terephthalic acid (TPA) as shown in Table 1 below.

Example 5

A recycled terephthalic acid monomer composition and a recycled plastic were prepared in the same manner as in Example 1, except that the recrystallization solvent in Example 1(1) was changed to dimethylacetamide (DMAc) in an amount of five times relative to that of the obtained terephthalic acid (TPA) as shown in Table 1 below.

Example 6

(1) Preparation of recycled terephthalic acid monomer composition

A recycled terephthalic acid monomer composition was prepared in the same manner as in Example 1 (1).

(2) Preparation of recycled plastic

300 g of the recycled terephthalic acid monomer composition obtained in Example 1(1) and 300 g of 1,4-butylene glycol were put into an esterification reactor, and 0.1 wt% of a TBT catalyst was added thereto. The mixture was reacted while maintaining 200-240°C 120~180 minutes, and the reaction was terminated when the reaction rate (when the amount of water, which is the reaction effluent, converted to the reaction rate) was 90% or more to obtain an oligomer.

Then, the prepared oligomer was transferred to a polycondensation reactor, the pressure was reduced from 760torr to 0.3torr for 30 minutes while maintaining 230 ~ 260°C, and the melt polycondensation was carried out, and the melt polycondensation reaction was carried out under high vacuum conditions of 0.3 torr or less until the torque applied to the stirrer reached a desired torque value. After completion of the reaction, it was discharged using nitrogen pressure, stranded, cooled and then pelletized to prepare a polybutylene terephthalate (PBT) resin.

<Comparative Example>

Comparative Example 1

A recycled terephthalic acid monomer composition and a recycled plastic were prepared in the same manner as in Example 1, except that in Example 1(1), the washing solvent was changed to a 1:1 mixed solution of water and NMP (10 times relative to the amount of terephthalic acid (TPA)), and washed at 20 ~ 30°C as shown in Table 1 below.

Comparative Example 2

A recycled terephthalic acid monomer composition and a recycled plastic were prepared in the same manner as in Example 1, except that in Example 1(1), washing was carried out two times at 20~30 °C with water in an amount of 10 times relative to that terephthalic acid (TPA), and recrystallization was not carried out as shown in Table 1 below.

Comparative Example 3

A recycled terephthalic acid monomer composition and a recycled plastic were prepared in the same manner as in Example 3, except that in Example 3(1), the washing temperature was changed to 20 ~ 30°C as shown in Table 1 below.

<Experimental Example 1>

The physical properties of the recycled terephthalic acid monomer compositions obtained in Examples and Comparative Examples were measured by the following methods, and the results are shown in Table 1 below.

1. Yield of terephthalic acid (TPA)

The yield of terephthalic acid (TPA) was calculated by the following Equation.

[Equation]

TPA yield (%) = {(Number of moles of TPA contained in the recycled terephthalic acid monomer composition) / (Number of moles of PET initially added)} X 100.

2. Particle size of terephthalic acid (TPA)

The maximum diameter and the minimum diameter of the terephthalic acid (TPA) particles contained in the recycled terephthalic acid monomer composition obtained in Examples and Comparative Examples were respectively measured through FE-SEM images with HITACHI-S4800.

3. Content of recrystallization organic solvent

5~20 mg of the recycled terephthalic acid monomer composition was collected as a sample under the conditions of normal pressure and 20 ~ 30°C, and dissolved in 1 ml of DMSO-d6 solvent, and then 1H NMR spectra were obtained using an Agilent DD1 500 MHz NMR instrument. All detected material peaks such as terephthalic acid (TPA), N-methyl-2-pyrrolidone (NMP), and dimethylacetamide (DMAc) were respectively designated and integrated using analysis software (MestReC). Based on the peak integral value, the weight ratio (ppm) of the recrystallization organic solvent N-methyl-2-pyrrolidone (NMP) and dimethylacetamide (DMAc) contained in 100 wt% of the total recycled terephthalic acid monomer composition analyzed in the sample was calculated.

4. Content of terephthalic acid (TPA) and isophthalic acid (IPA)

5~20 mg of recycled terephthalic acid monomer composition was collected as a sample under the conditions of normal pressure and 20 ~ 30 °C, dissolved in 1 ml DMSO-d6 solvent, and then 1H NMR spectrum was obtained through an Agilent DD1 500 MHz NMR instrument. All material peaks detected, such as terephthalic acid (TPA) and isophthalic acid (IPA), were respectively assigned and integrated using an analysis software (MestReC). The molar ratio (mol%) of terephthalic acid and isophthalic acid contained in 100 mol% of all monomer compounds analyzed in the sample was calculated based on the peak integral value.

[Table 1]

| Measurement result of Experimental Example 1 | | | | | | | |
|---|---|---|---|---|---|---|---|
| Category | Recrystallizatio n condition | Washing conditio n | Yiel d (%) | Particle size ( $\mu$m) | Recrystallizatio n organic solvent (ppm) | TPA (mol% ) | IPA (mol% ) |
| Example 1 | NMP / 10 times | 205 °C / water / 10 times | 60.4 | 100-300 | 0 | 100.0 | 0.0 |
| Example 2 | NMP / 5 times | 205 °C / water / 10 times | 67.9 | 60-150 | 0 | 100.0 | 0.0 |
| Example 3 | NMP / 4 times | 205 °C / water / 10 times | 88.6 | 40-70 | 0 | 100.0 | 0.0 |
| Example 4 | NMP / 4 times | 205 °C / water / 5 times | 69.6 | 10-20 | 0 | 99.9 | 0.1 |

(continued)

| Measurement result of Experimental Example 1 | | | | | | | |
|---|---|---|---|---|---|---|---|
| Category | Recrystallization condition | Washing condition | Yield (%) | Particle size ($\mu$m) | Recrystallization organic solvent (ppm) | TPA (mol%) | IPA (mol%) |
| Example 5 | DMAc / 5 times | 205 °C / water / 10 times | 85.0 | 10-15 | 0 | 99.9 | 0.1 |
| Example 6 | Same as Example 1 | | | | | | |
| Comparative Example 1 | NMP / 10 times | 20-30°C / water +NMP / 10 times | 43.6 | 5-10 | 500 | 99.2 | 0.8 |
| Comparative Example 2 | - | 20-30 °C / water / 10 times | 94.8 | 0.5-1 | 0 | 97.8 | 2.2 |
| Comparative Example 3 | NMP / 4 times | 20-30°C / water / 10 times | 25.0 | 0.5-1 | 400 | 98.8 | 1.2 |

As shown in Table 1 above, 99.9 mol%~100 mol% of the monomers contained in the recycled terephthalic acid monomer composition obtained in Examples 1 to 5 contained terephthalic acid, so that isophthalic acid, which is an impurity, was completely removed, and the recrystallization organic solvent content, which is other impurity, was not detected as 0 ppm, indicating high purity terephthalic acid purification efficiency. On the other hand, it was confirmed that in the monomers contained in the recycled terephthalic acid monomer composition obtained in Comparative Example 1, isophthalic acid as an impurity was contained at 0.8 mol%, which was excessive relative to Examples, the content of the recrystallization organic solvent as another impurity was 500 ppm, which was excessive compared to Examples, and thus, the purification efficiency of terephthalic acid was greatly reduced.

In addition, it could be confirmed that in the monomers contained in the recycled terephthalic acid monomer composition obtained in Comparative Example 2, isophthalic acid as an impurity was contained at 2.2 mol%, which was excessive relative to Examples, and the terephthalic acid purification efficiency was greatly reduced.

Further, in the monomers contained in the recycled terephthalic acid monomer composition obtained in Comparative Example 3, isophthalic acid as an impurity was contained at 1.2 mol% compared to Examples, which was excessive compared to Examples, and the content of the recrystallization organic solvent as another impurity was 400 ppm, which was excessive compared to Examples, so that the terephthalic acid purification efficiency was greatly reduced.

On the other hand, the particle size of the terephthalic acid contained in the recycled terephthalic acid monomer composition obtained in Examples was 10 $\mu$m to 300 $\mu$m, which was increased compared to Comparative Example whose particle sizes were 0.5 $\mu$m to 10 $\mu$m.

<Experimental Example 2>

The physical properties of the recycled plastics obtained in the Examples and Comparative Examples were measured by the following methods, and the results are shown in Table 2 below.

1. Melt Flow Index (MFI)

(1) The melt flow index of the thermoplastic polyester elastomers (TPEE) obtained in Examples 1 to 5 and Comparative Examples 1 to 3 was measured at 230°C and a load of 2.16 kg for 4 minutes according to ASTM D1238.
(2) The melt flow index of polybutylene terephthalate (PBT) obtained in Example 6 was measured at 250°C and a load of 2.16 kg for 4 minutes according to ASTM D1238.

[Table 2]

| Measurement result of Experimental Example 2 | | | | |
|---|---|---|---|---|
| Category | Polymer | Recrystallization condition | Washing condition | MFI (g/10min) |
| Example 1 | TPEE | NMP / 10 times | 205°C / water / 10 times | 21.0 |

(continued)

| Measurement result of Experimental Example 2 | | | | |
|---|---|---|---|---|
| Category | Polymer | Recrystallization condition | Washing condition | MFI (g/10min) |
| Example 2 | TPEE | NMP / 5 times | 205°C / water / 10 times | 19.5 |
| Example 3 | TPEE | NMP / 4 times | 205°C / water / 10 times | 19.6 |
| Example 4 | TPEE | NMP / 4 times | 205°C / water / 5 times | 19.9 |
| Example 5 | TPEE | DMAc / 5 times | 205°C / water / 10 times | 26.0 |
| Example 6 | PBT | NMP / 10 times | 205°C / water / 10 times | 20.2 |
| Comparative Example 1 | TPEE | NMP / 10 times | 20-30°C / water + NMP / 10 times | 20.5 |
| Comparative Example 2 | TPEE | - | 20-30°C / water / 10 times | 43.9 |
| Comparative Example 3 | TPEE | NMP / 4 times | 20-30°C / water / 10 times | 23.5 |

As shown in Table 2, it was found that in the case of the TPEE (co)polymer synthesized from the recycled terephthalic acid monomer composition obtained in Examples 1 to 5, the melt flow index was 19.5 g/10 min to 26 g/10 min. In addition, it was found that in the case of the PBT (co)polymer synthesized from the recycled terephthalic acid monomer composition obtained in Example 6, the melt flow index was 20.2 g/10 min.

On the other hand, in the case of the TPEE (co)polymer synthesized from the recycled terephthalic acid monomer composition obtained in Comparative Example 2, the melt flow index was greatly increased to 43.9 g/10 min, confirming that it is defective.

## Claims

1.  A monomer composition for synthesizing recycled plastic,

    which comprises terephthalic acid, and
    which further comprises isophthalic acid having a molar ratio of less than 0.5 mol% based on 100 mol% of the monomer composition for synthesizing recycled plastic,
    wherein the terephthalic acid has a particle size of 10 $\mu$m or more and 300 $\mu$m or less, and
    wherein the monomer composition for synthesizing recycled plastic is recovered from a (co)polymer synthesized from a monomer containing terephthalic acid.

2.  The monomer composition for synthesizing recycled plastic according to claim 1, wherein:
    the terephthalic acid and isophthalic acid are recovered from the (co)polymer that is synthesized from the monomer containing terephthalic acid used for the recovery of the monomer composition for synthesizing recycled plastic.

3.  The monomer composition for synthesizing recycled plastic according to claim 1, wherein:
    the terephthalic acid is contained in an amount of more than 99.5 mol% based on 100 mol% of the total monomer compounds contained in the monomer composition for synthesizing recycled plastics.

4.  The monomer composition for synthesizing recycled plastic according to claim 1,
    which further comprises an organic solvent having a weight ratio of less than 10 ppm based on 100% by weight of the monomer composition for synthesizing recycled plastic.

5.   The monomer composition for synthesizing recycled plastic according to claim 4, wherein:
    the organic solvent comprises N-methyl-2-pyrrolidone or dimethylacetamide.

6.  The monomer composition for synthesizing recycled plastic according to claim 1, wherein:
    the (co)polymer synthesized from the monomer containing terephthalic acid comprises at least one (co)polymer selected from the group consisting of polyalkylene terephthalate, polyalkylene terephthalate-based copolymer, and thermoplastic polyester elastomer.

7. A method for preparing a monomer composition for synthesizing recycled plastic, comprising the steps of:

subjecting a (co)polymer synthesized from a monomer containing terephthalic acid to a depolymerization reaction and removing a diol component;
dissolving a depolymerization reaction product from which the diol component has been removed in an organic solvent and then recrystallizing the depolymerization reaction product; and
washing the result of the recrystallization step with a solvent at a temperature of 200°C or more and 300°C or less.

8. The method for preparing a monomer composition for synthesizing recycled plastic according to claim 7, wherein:
the solvent used in the washing step comprises water.

9. The method for preparing a monomer composition for synthesizing recycled plastic according to claim 7, wherein:
in the washing step, 5 parts by weight or more and 10 parts by weight or less of the solvent is used based on 1 part by weight of the result of the recrystallization step.

10. The method for preparing a monomer composition for synthesizing recycled plastic according to claim 7, wherein:
the organic solvent used in the recrystallization step comprises N-methyl-2-pyrrolidone or dimethylacetamide.

11. The method for preparing a monomer composition for synthesizing recycled plastic according to claim 7, wherein:
in the recrystallization step, 4 parts by weight or more and 10 parts by weight or less of the organic solvent is used based on 1 part by weight of the depolymerization reaction product from which the diol component has been removed.

12. The method for preparing a monomer composition for synthesizing recycled plastic according to claim 7, wherein:

the recrystallization step comprises,
dissolving the depolymerization reaction product from which the diol component has been removed in the organic solvent at a temperature of 50°C or more and 130°C or less; and
cooling a solution in which the depolymerization reaction product dissolved to a temperature of 10°C or less.

13. The method for preparing a monomer composition for synthesizing recycled plastic according to claim 7, wherein:
the depolymerization reaction of the (co)polymer synthesized from the monomer containing terephthalic acid is carried out by reacting a base in an amount of 1.5 moles or more and 2.5 moles or less relative to 1 mole of the (co)polymer synthesized from the monomer containing terephthalic acid.

14. The method for preparing a monomer composition for synthesizing recycled plastic according to claim 7, wherein:
the depolymerization reaction of the (co)polymer synthesized from the monomer containing terephthalic acid is carried out in the presence of an alkylene glycol solvent.

15. The method for preparing a monomer composition for synthesizing recycled plastic according to claim 7, wherein:

before the step of dissolving the depolymerization reaction product from which the diol component has been removed in the organic solvent and then recrystallizing the depolymerization reaction product,
the method further comprises subjecting the depolymerization reaction product from which the diol component has been removed to a neutralization reaction with an acid.

16. The method for preparing a monomer composition for synthesizing recycled plastic according to claim 7, wherein:

after the step of subjecting the (co)polymer synthesized from the monomer containing terephthalic acid to a depolymerization reaction and removing the diol component,
the method further comprises purifying the depolymerization reaction product from which the diol component has been removed.

17. A recycled plastic comprising a reaction product of the monomer composition for synthesizing recycled plastic of claim 1 and a comonomer.

**18.** The recycled plastic according to claim 17, wherein:
the recycled plastic has a melt flow index of 15 g/10 min or more and 27 g/10 min or less as measured by ASTM D1238.

**19.** A molded product comprising the recycled plastic of claim 17.

**20.** A plasticizer composition comprising a reaction product of the monomer composition for synthesizing recycled plastic of claim 1 and an alcohol.

## INTERNATIONAL SEARCH REPORT

| International application No. |
| --- |
| **PCT/KR2022/010302** |

**A. CLASSIFICATION OF SUBJECT MATTER**

**C08J 11/24**(2006.01)i; **C07C 63/26**(2006.01)i; **C07C 51/09**(2006.01)i; **C07C 51/42**(2006.01)i; **C08G 63/181**(2006.01)i; **C08G 63/183**(2006.01)i; **C08K 5/00**(2006.01)i; **C08K 5/10**(2006.01)i

According to International Patent Classification (IPC) or to both national classification and IPC

**B. FIELDS SEARCHED**

Minimum documentation searched (classification system followed by classification symbols)

C08J 11/24(2006.01); C07C 51/09(2006.01); C07C 51/42(2006.01); C08G 63/78(2006.01); C08J 11/04(2006.01); C08J 11/10(2006.01); C08J 11/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 재활용 플라스틱(recycled plastic), 폴리에틸렌테레프탈레이트(polyethylene terephthalate), 테레프탈산(terephthalic acid), 이소프탈산(isophthalic acid), 해중합(depolymerization), 입자 직경(particle diameter)

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2018-0027539 A (LOOP INDUSTRIES, INC.) 14 March 2018 (2018-03-14) See claims 1, 9 and 12-15; paragraphs [0004], [0005], [0018], [0019], [0021], [0028], [0032], [0041], [0043], [0052], [0062], [0067], [0078], [0092], [0114], [0118], [0143], [0145]-[0148], [0150] and [0166]; and examples 1 and 5. | 1-4,6,17-19 |
| Y | | 5,7-16,20 |
| Y | KR 10-2002-0078366 A (KOREA RESEARCH INSTITUTE OF CHEMICAL TECHNOLOGY) 18 October 2002 (2002-10-18) See paragraphs [0004], [0005] and [0019]. | 5,7-16 |
| Y | KR 10-2013-0130877 A (BP CORPORATION NORTH AMERICA INC.) 02 December 2013 (2013-12-02) See paragraph [0054]. | 20 |

☑ Further documents are listed in the continuation of Box C.     ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **07 November 2022** | **07 November 2022** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/010302**

| C. | DOCUMENTS CONSIDERED TO BE RELEVANT | | |
|---|---|---|---|
| Category* | Citation of document, with indication, where appropriate, of the relevant passages | | Relevant to claim No. |
| A | WO 2007-148353 A1 (COBARR S.P.A.) 27 December 2007 (2007-12-27)<br>See entire document. | | 1-20 |
| A | WO 2013-025186 A1 (EASTMAN CHEMICAL COMPANY) 21 February 2013 (2013-02-21)<br>See entire document. | | 1-20 |

Form PCT/ISA/210 (second sheet) (July 2022)

**INTERNATIONAL SEARCH REPORT**
Information on patent family members

International application No.

**PCT/KR2022/010302**

| Patent document cited in search report | | | Publication date (day/month/year) | Patent family member(s) | | | Publication date (day/month/year) |
|---|---|---|---|---|---|---|---|
| KR | 10-2018-0027539 | A | 14 March 2018 | AR | 105289 | A1 | 20 September 2017 |
| | | | | AU | 2016-290108 | A1 | 08 February 2018 |
| | | | | AU | 2016-290108 | B2 | 06 February 2020 |
| | | | | BR | 112018000128 | A2 | 04 September 2018 |
| | | | | CA | 2991515 | A1 | 12 January 2017 |
| | | | | CN | 108093634 | A | 29 May 2018 |
| | | | | CN | 108093634 | B | 28 May 2021 |
| | | | | DK | 3320033 | T3 | 31 May 2021 |
| | | | | EA | 034835 | B1 | 26 March 2020 |
| | | | | EA | 201890164 | A1 | 31 August 2018 |
| | | | | EP | 3320033 | A1 | 16 May 2018 |
| | | | | EP | 3320033 | B1 | 10 March 2021 |
| | | | | ES | 2873153 | T3 | 03 November 2021 |
| | | | | HR | P20210831 | T1 | 06 August 2021 |
| | | | | HU | E054516 | T2 | 28 September 2021 |
| | | | | IL | 256731 | A | 29 March 2018 |
| | | | | IL | 256731 | B | 27 February 2020 |
| | | | | JP | 2018-522107 | A | 09 August 2018 |
| | | | | JP | 2021-073332 | A | 13 May 2021 |
| | | | | JP | 6876332 | B2 | 26 May 2021 |
| | | | | JP | 7058024 | B2 | 21 April 2022 |
| | | | | LT | 3320033 | T | 25 June 2021 |
| | | | | MX | 2018000320 | A | 28 May 2018 |
| | | | | PH | 12018500043 | A1 | 09 July 2018 |
| | | | | PL | 3320033 | T3 | 08 November 2021 |
| | | | | PT | 3320033 | T | 02 June 2021 |
| | | | | RS | 61918 | B1 | 30 June 2021 |
| | | | | SI | 3320033 | T1 | 30 July 2021 |
| | | | | TW | 201710341 | A | 16 March 2017 |
| | | | | TW | I610970 | B | 11 January 2018 |
| | | | | US | 10087130 | B2 | 02 October 2018 |
| | | | | US | 10640442 | B2 | 05 May 2020 |
| | | | | US | 2017-0008826 | A1 | 12 January 2017 |
| | | | | US | 2017-0152203 | A1 | 01 June 2017 |
| | | | | US | 2018-0370894 | A1 | 27 December 2018 |
| | | | | US | 9550713 | B1 | 24 January 2017 |
| | | | | WO | 2017-007965 | A1 | 12 January 2017 |
| | | | | ZA | 201800357 | B | 26 June 2019 |
| KR | 10-2002-0078366 | A | 18 October 2002 | KR | 10-0429385 | B1 | 29 April 2004 |
| KR | 10-2013-0130877 | A | 02 December 2013 | AR | 058867 | A1 | 27 February 2008 |
| | | | | AR | 094273 | A2 | 22 July 2015 |
| | | | | CA | 2626033 | A1 | 05 July 2007 |
| | | | | CA | 2626033 | C | 03 September 2013 |
| | | | | CA | 2820149 | A1 | 05 July 2007 |
| | | | | CN | 101351494 | A | 21 January 2009 |
| | | | | EP | 2292684 | A2 | 09 March 2011 |
| | | | | EP | 2292684 | A3 | 20 April 2011 |
| | | | | EP | 2752446 | A1 | 09 July 2014 |
| | | | | JP | 2009-522305 | A | 11 June 2009 |
| | | | | JP | 2014-094955 | A | 22 May 2014 |

Form PCT/ISA/210 (patent family annex) (July 2022)

**INTERNATIONAL SEARCH REPORT**
Information on patent family members

International application No.

**PCT/KR2022/010302**

| Patent document cited in search report | | | Publication date (day/month/year) | Patent family member(s) | | | Publication date (day/month/year) |
|---|---|---|---|---|---|---|---|
| | | | | JP | 5491033 | B2 | 14 May 2014 |
| | | | | KR | 10-2008-0091453 | A | 13 October 2008 |
| | | | | US | 2009-0171113 | A1 | 02 July 2009 |
| | | | | WO | 2007-076384 | A2 | 05 July 2007 |
| | | | | WO | 2007-076384 | A3 | 23 August 2007 |
| WO | 2007-148353 | A1 | 27 December 2007 | BR | PI0621743 | A2 | 20 December 2011 |
| | | | | CN | 101484409 | A | 15 July 2009 |
| | | | | CN | 101484409 | B | 05 September 2012 |
| | | | | EP | 2038246 | A1 | 25 March 2009 |
| | | | | KR | 10-2009-0024799 | A | 09 March 2009 |
| | | | | MX | 2008016450 | A | 22 January 2009 |
| | | | | US | 2009-0287017 | A1 | 19 November 2009 |
| WO | 2013-025186 | A1 | 21 February 2013 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210094470
- KR 1020210094471
- KR 1020210094472
- KR 1020210094473